# EUROPEAN PATENT SPECIFICATION

(11) **EP 4 115 918 B1**
(45) Date of publication and mention of the grant of the patent: **12.11.2025**
(21) Application number: 22183306.4
(22) Date of filing: 06.07.2022
(51) Int. Cl.: A61M 1/00, A61M 5/31, A61M 5/32, A61B 17/00

(54) **SYRINGE SYSTEM FOR PROCESSING A FLUID**
SPRITZENSYSTEM ZUR VERARBEITUNG EINES FLUIDS
SYSTÈME DE SERINGUE POUR LE TRAITEMENT D'UN FLUIDE

(30) Priority: 06.07.2021 EP 21183942
(43) Date of publication of application: 11.01.2023
(73) Proprietor: Prof. Dr. Dr. Prantl, Dr. Baringer, Dr. Felthaus Und Eigenberger GbR (REG-2), 93051 Regensburg (DE)
(72) Inventor: Prantl, Lukas, 93051 Regensburg (DE); Baringer, Magnus, 93077 Bad Abbach (DE); Felthaus, Oliver, 93077 Bad Abbach (DE); Eigenberger, Andreas, 93053 Regensburg (DE)
(74) Representative: Drexl, Janna

(56) References cited:
- US-A1- 2011 224 610
- US-A1- 2016 081 878
- US-A1- 2017 203 040
- US-A1- 2017 368 226
- US-A1- 2021 130 783

## Description

The present invention refers to a device for processing a fluid by centrifugation and/or sedimentation comprising: a syringe body (1) a syringe plunger (2) comprising a sealing plate (6) and a perforated sealing plate (7) at the lower end of the plunger, wherein the perforated sealing plate (7) is movably attached to the syringe plunger (2) and the distance between the movable perforated sealing plate (7) and the sealing plate (6) is variable, and a syringe closure (3), comprising at least one perforation cage (9). The present invention also refers to a syringe plunger (2) and a syringe closure (3) of a device according to the present invention. Moreover, the present invention refers to a method of processing a fluid. In addition, the present invention refers to a use of the device, the syringe plunger (2), the syringe closure (3) or the method according to the present invention for the clean and lossless release of individual phases of multiphase fluids after separation using centrifugal or gravitational forces, in particular for separating a fluid into two, three or more phases, for enriching cells in a fat graft or bone marrow, for homogenizing a fat graft and/or for processing whole blood.

Fat transfer (lipofilling, fat grafting) is one of the most common procedures in plastic surgery, which is used for the treatment of age-, disease- or trauma-related soft tissue volume deficits. The operative procedure usually includes the harvesting of the fat, usually performed by liposuction, the processing, and the application. All steps are performed during the same intervention. Although, optimizing the methods for lipofilling was focused by plastic surgeons for years, the volume loss of the lipo-transplant still varies up to 70 %.

To support a better bloodless harvesting and a higher viability of the fat graft, tumescent solution should be injected before and during the liposuction. The use of tumescent solution, which can differ in the exact composition like local anaesthetics, is considered standard of care as well. However, all beneficial components, like grow factors, vital adipocytes, progenitor cells of the stromal vascular fraction (SVF) and adipose stem cells are in the fat graft. Hence, to optimize the outcome of lipofilling, the tumescent solution and the blood should be removed during the processing step. Moreover, lipids released by disrupted adipocytes should be also removed. Based on different densities, the heavier tumescent solution with the blood contamination sediments immediately after the suction. To speed up this progress and to ensure a higher level of purity, the lipoaspirate can be centrifuged.

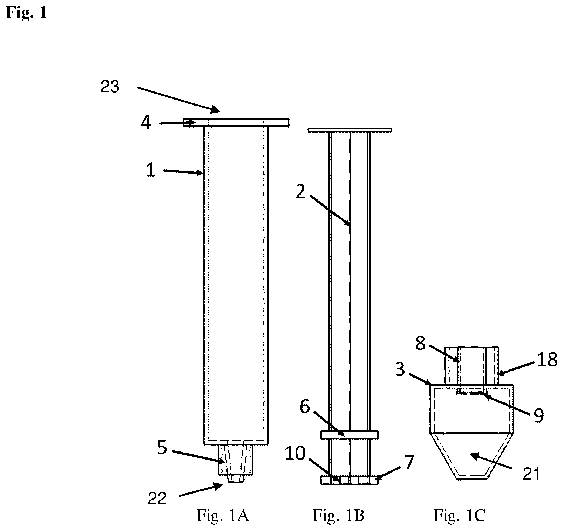

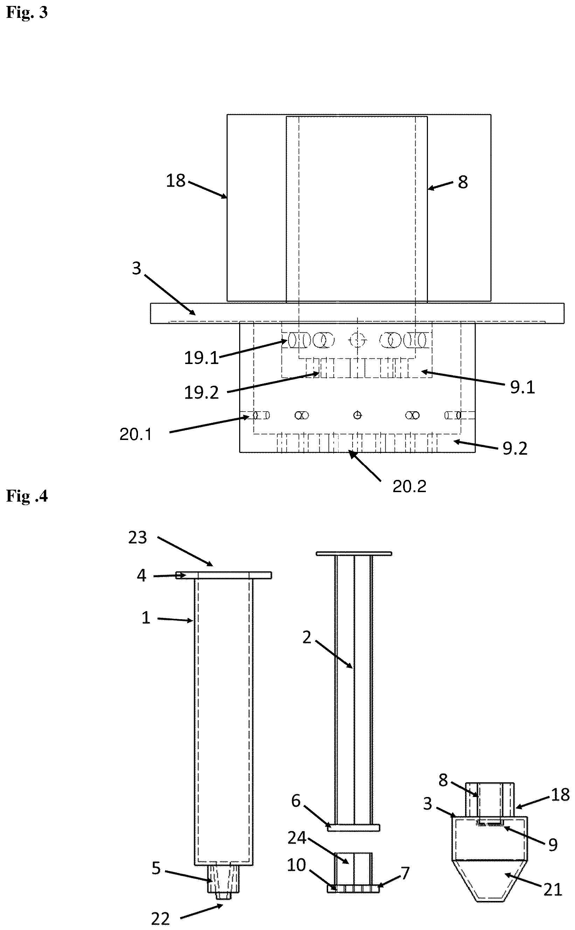

After the centrifugation three different phases, the tumescent solution/ tissue fluid, the pure concentrated fat graft and an oily phase, containing the released lipids, can be detected. However, the separated phases remain in the same syringe and the undesired phases must be removed manually. Therefore, the tumescent phase with the blood contamination is removed by pressing the syringe plunger until the fat graft reaches the opening (e.g. in form of a luer). Afterwards, the fat graft is transferred to another syringe using a luer-to-luer connector. Due to different viscosities, the transfer leads to a remixing of the fat- and the oily phase at the phaseboundary. The remaining mixture in the first syringe has inferior quality, should not be used for lipofilling and has to be discarded together with the pure oily phase. These manual steps to separate the fat graft will be eliminated by using the present invention, which is seen in Figure 1 and Figure 4. These manual steps are time consuming and present risks for contamination, infection, tissue damage and imprecise separation.

After the processing described above, the fat graft could be transplanted. However, there are some applications where the fat graft lobule size must be minimized and an enrichment of progenitor cells is desirable. This so called micro- or nanofat is used if transplantation must be performed with small sized cannulas, like face lipofilling. Furthermore, it is discussed if micro- or nanofat can increase the take rate of the transplant by enrichment of the progenitor cells. Therefore, the syringe containing the one-time centrifuged fat graft is connected to an empty syringe by a special luer to luer connector. This connector usually has a small variable inner diameter like 0.2 mm to 2.0 mm. The fat graft is pushed through this connector for a defined number of times. Due to shear forces during intersyringe processing with reduction of the inner diameter of the connector the size of large-scaled adipose tissue particles decreases. This shear force also disrupts mature adipocytes, releasing their lipids. Hence, a second centrifugation step after the intersyringe processing should be done to separate the lipids from the concentrated fat graft. As described in Prantl et al. ("Shear Force Processing of Lipoaspirates for Stem Cell Enrichment Does Not Affect Secretome of Human Cells Detected by Mass Spectrometry In Vitro". Journal Plastic and Reconstructive Surgery, 2020) during CELT (Cell Enriched Lipo-Transfer), the separation of the cell enriched fraction after the centrifugation is performed by manual steps and should be optimized with the syringe system provided by the present invention.

Notwithstanding the processing described above, there are several different ways to process the lipoaspirate for lipofilling / fat transfer procedures. For example, there is the LipoCollector (manufacturer: HumanMed) or similar systems as e.g. described in WO 2012/148502 A3 where the lipo-graft and the tumescent solution are separated in a container by sedimentation and a mesh insert. Another example is the Revolve System by LifeCell Corporation or a system described in AU 2017/203362 A1 for harvesting, filtering and transferring of adipose tissue. To optimize the outcome of lipofilling procedures, Cell Assisted Lipo-Transfer (CAL) is performed sometimes. During CAL, fat graft is processed by enzymatic digestion to isolate the Stromal Vascular Fraction (SVF) which is afterwards added to non-processed fat graft to increase the concentration of adipose stem cells in the lipo-transplant. To support CAL the Q-Graft device by Human Med or similar systems as described in CA 2938268 A1 can be used. However, this and similar procedures are time-consuming, expensive, and strongly regulated because of the use of xenogeneic enzyme products. Therefore, there is still a need for a medical device for supporting the processing of small-volume lipo-transplants by optimizing the outcome by mechanical cell enrichment, eliminating manual processing steps and the risks of contamination/infection and reducing the processing time. There is also a need for a device which can be used also for pre-processing as e.g. for collagenase digestion.

The knowledge of the benefits of lipoaspirate processing to reduce tissue particle size has led to previous inventions to help with this task. These, however, focus on the processing part. In contrast, the present invention focusses on the processing, the separation of the single phases obtained by in the processing step and the release of the separated phases. For example, the device described in US 2021/0130783 A1 serves the purpose of tissue shredding, only, and is neither concerned with the separation of phases nor the release of separated phases. The devices described in US 2017/0203040 A1 and US 2006/0093527 A1 are about phase separation. However, these devices achieve this separation through the application of filter and a vacuum source, not by centrifugal or gravitational forces, which leads to a different product. Moreover, the devices described in the prior art are not concerned with the lossless and efficient release of the separated phases.

The problem to be solved by the present invention was thus to gain a higher level of purity by exactly separating and storing the different phases in reservoirs. Therefore, no remixing of the phases during following processing steps, like mechanical enrichment of stem cells during CELT, can be observed. In particular, the oil phase can be separated precisely when transferring the fat phase. Increasing the purity of the fat-phase leads to increased vital cells concentration, like stem cells, in the transplant and hence, leads to an increased take rate of the transplant. Since only a limited volume can be transplanted, the purity of the fat phase and the concentration of stem cells is of particular importance for engraftment. A further problem to be solved by the present invention is the provision of a syringe system for minimizing the number of needed manual work steps and minimizing the risks for contamination, infection, tissue damage and imprecise separation. Still a further problem to be solved by the present invention is the provision of a system for processing lipoaspirate which is more cost- and time efficient. In particular, the problem to be solved by the present invention is to provide a system which can be used for large and small-volume lipo-transplants. A further problem to be solved is the provision of a syringe system and method for the clean and lossless release of individual phases of multiphase fluids after separation using centrifugal or gravitational forces. Another problem to be solved is the provision of only one device in which both the processing and the centrifugation can be performed while undesired commingling of the separated phases at the phase boundaries during the release of the separated phases is prevented.

Although the design of the invention is optimized to separate lipoaspirate from the tumescence/blood/water phase and the oily phase for fat grafting, the inventors of the present invention have found that it can also be utilized to process further fluids such as samples obtained by a subject as e.g. for preparing a platelet concentrate (PRP, platelet rich plasma) from whole blood. PRP is obtained by centrifugation of whole blood in order to separate the red blood cells (erythrocytes) from platelets (thrombocytes) and white blood cells (leukocytes). Thrombocytes, the desired part of the whole blood when preparing PRP, are rich in growth factors and can be stimulated to release these factors. After centrifugation the red blood cell phase is the undermost phase, the cell free plasma is the uppermost phase. In between is the buffy coat, a thin layer containing the leukocytes and thrombocytes. Because the invention is designed to separate phases depending on density, this device can be used to obtain PRP as well. The undesired erythrocyte phase is collected in the collection cup of the syringe closure. Depending on the size of the openings of the perforated sealing plate of the plunger the buffy coat containing the growth factorrich platelets can be utilized with the full plasma volume or the plasma is collected in the space between the plunger plates and a highly concentrated PRP can be utilized. This is especially useful when PRP is combined with the cell enriched lipograft to even further support tissue regeneration.

The problem underlying the present invention is solved by the subject matter defined in the claims.

The following figures serve to illustrate the invention.
Figure 1 depicts a device or syringe system according to a preferred embodiment comprising a syringe body (1) (in an upright position), a syringe plunger (2) (in an upright position) and a syringe closure (3) (in an upright position). The syringe body (1) comprises a luer-lock connection (5) at its lower end and a finger flange (4) at its upper end. The syringe plunger (2) comprises a sealing plate (6) and a perforated sealing plate (7) which comprises several openings (10). Said perforated sealing plate (7) is preferably movably attached to the syringe plunger (2) so that it is freely movable between the lower end of the plunger and the sealing plate (6). The syringe closure (3) comprises a female luer (8), a perforation cage (9), a collection cup (21) and a force absorption ring (18).

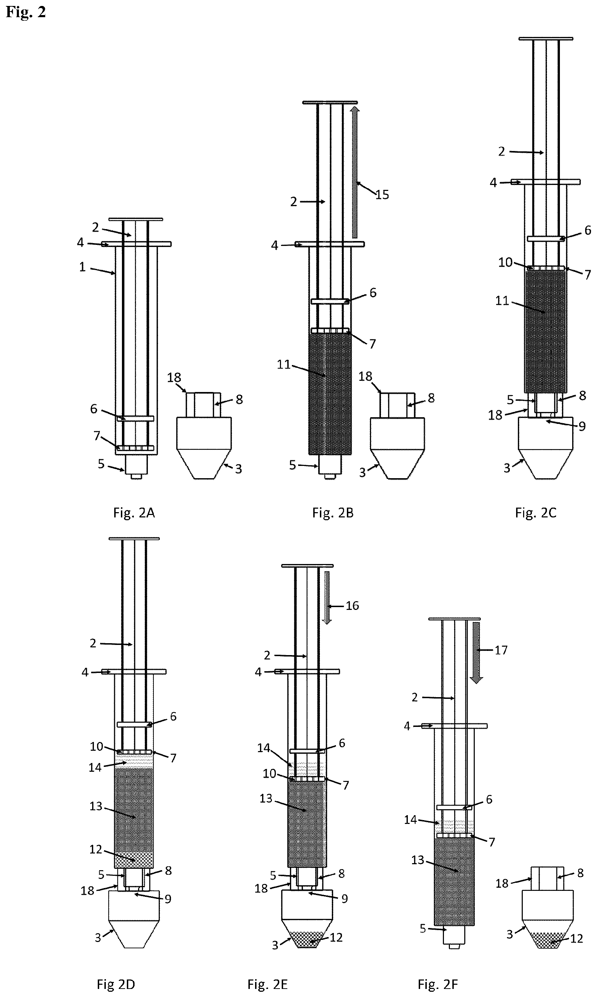

Figure 2 shows in Figs. 2A to 2F the different steps of processing lipoaspirate with an embodiment of a preferred syringe system. In Fig. 2A the syringe plunger (2) as described in Figure 1 is inserted into a syringe body (1) as described in Figure 1. In addition, the syringe closure (3) is shown. Fig. 2B shows the same arrangement as shown in Fig. 2A but after applying a force (15) on the syringe plunger (2) for filling the syringe body (1) with lipoaspirate (11). Fig. 2C shows the syringe system comprising lipoaspirate (11) after the syringe closure (3) has been attached to the syringe body (1) by use of a male (5) and female (8) luer-lock. Fig. 2D corresponds to Fig. 2C, wherein the lipoaspirate is separated into its three phases, namely the oily phase (14), the fat graft phase (13) and the tumescent phase (12) by sedimentation. Fig. 2E corresponds to Fig. 2D after application of a manual pressure (16) by which the tumescent phase (12) has been pushed into the collection cup of the syringe closure (3) and the perforated sealing plate (preferably movable) through the oily phase (14). In case of accelerating the sedimentation by centrifugation, there is a continuous transition from Fig. 2C to Fig. 2E, because during the separation of the lipoaspirate (11) into the oily- (14), fat (13) and tumescent-phase the centrifugal force (16) leads to an immediate passing into the syringe closure (3) and the reservoir between the syringe plates (6 and 7). Figure 2F corresponds to Figure 2E after disconnecting the syringe closure (3) from the syringe body (1). In Fig. 2F the fat graft phase (13) is ready for being used directly in lipofilling or for further processing such as minimizing the fat graft lobule size each by applying a force (17) to the syringe plunger (2).
Figure 3 displays a preferred embodiment of the upper part of the syringe closure (3) comprising a female luer (8), a force absorption ring (18), a first perforation cage (9.1) and a second perforation cage (9.2). The first perforation cage (9.1) comprises 5 vertical openings (19.2) and 10 horizontal openings (19.1). The second perforation cage (9.2) comprises 9 vertical openings (20.2) and 10 horizontal openings (20.1).
Figure 4 depicts a preferred embodiment of the device or syringe system as shown in Figure 1, showing that the perforated sealing plate (7) may be provided as an individual part comprising a vertical extension (24).

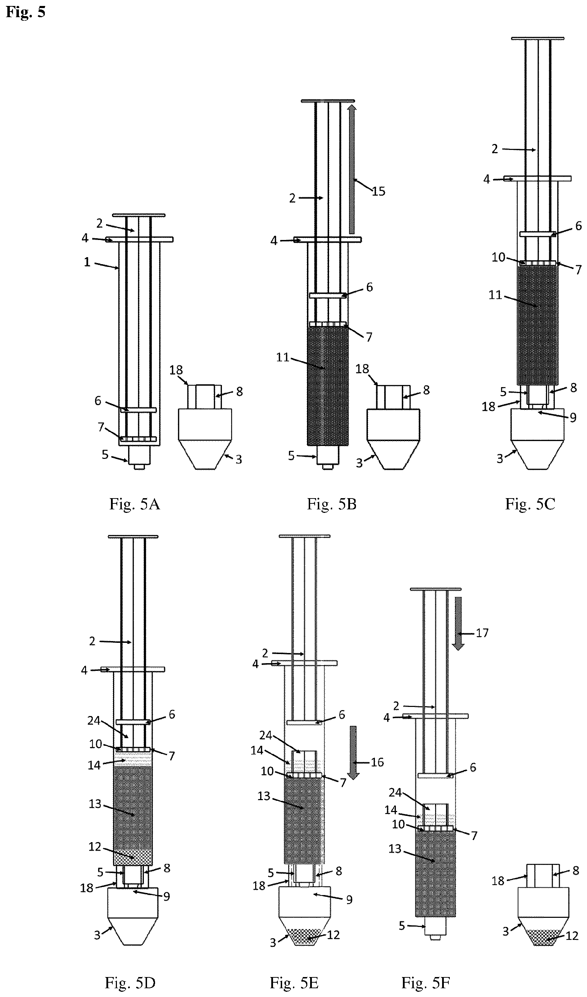

Figure 5 displays in Figs. 5A to 5F the different steps of processing lipoaspirate as already shown in Fig. 2 but with an embodiment of a preferred syringe system as shown in Figure 4. Figs 5A to 5F correspond to Figs 2A to 2F with the exception that the perforated sealing plate (7) with its extension (24) may form a separate lower part of the syringe plunger (2) as shown in Fig. 5E and 5F. The perforated sealing plate (7) with its extension (24) is preferably pressed through the oily phase (14) due to a centrifugal force (16).

The term "fluid" as used herein refers to any substance which can be processed in the device according to the present invention. Accordingly, said fluid is preferably a substance that continually deforms or flows under an applied shear stress or an external force. Preferably, the term fluid comprises liquids and suspension comprising a solid dispersed phase and a liquid dispersion medium. More preferably, the fluid is a mixture which can be separated into two, three or more phases by sedimentation and/or centrifugation. In a preferred embodiment of the present invention, the fluid is lipoaspirate, blood, in particular anticoagulated whole blood or coagulated whole blood, a fat graft or bone marrow.

The term "lipoaspirate" as used herein refers to the material removed by lipoaspiration or liposuction. During lipoaspiration or liposuction adipose tissue and tissue fluid are removed from an area of the body of an individual such as an animal, a mammal or a human. In particular, lipoaspirate comprises those components which can be used as a fat graft such as vital adipocytes, progenitor cells of the stromal vascular fraction (SVF), adipose stem cells and growth factors which can be used for lipofilling and for tissue regeneration. In addition, lipoaspirate comprises lipids released by disrupted adipocytes which should be removed prior to lipofilling. Usually a tumescent solution is injected before and during liposuction. Thus, lipoaspirate may not only comprise the body materials as described above but additionally also a tumescent solution comprising for example local anaesthetics and vasoconstrictors like adrenalin. If lipoaspirate is centrifugated it is separated in particular into three different phases:
(i) A first phase comprising tissue fluid, blood contamination and optional the tumescent solution. The first phase is the heaviest phase and thus the lowest phase of centrifugated lipoaspirate. The first phase is also called "tumescent phase" herein.
(ii) A second phase comprising the components for a fat graft, in particular cells (Adipose-derived Mesenchymal Stem/Stromal Cells (AD-MSCs), Hematopoietic stem cells (HSCs), Regulatory T cells (T_{reg} Cells), Adipocytes, Pericytes, Endothelial Cells (ECs), mast-cells, fibroblasts, dendritic cells, intra-adventitial smooth muscular-like cells), the extracellular matrix and growth factors. The second phase is lighter than the tumescent phase and thus above the tumescent phase of centrifugated lipoaspirate. The second phase is also called "fat graft phase", "fat graft" or "middle phase" herein.
(iii) A third phase comprising lipids e.g. released by disrupted adipocytes. The third phase is lighter than the first and the second phase and thus the top layer of sedimented and/or centrifugated lipoaspirate. The third phase is also called "oily phase" or "uppermost phase" herein.

The term "Platelet-rich plasma (PRP)" as used herein refers to a thrombocyte concentrate obtained from centrifuged whole blood with a higher platelet concentration than in whole blood. Platelets are rich in growth factors including EGF (epidermal growth factor), FGF (fibroblast growth factor), IGFs (insulin-like growth factors), PDGF (platelet-derived growth factor), VEGF (vascular endothelial growth factor), TGF-β (transforming growth factor beta), Interleukin 8, KGF (keratinocyte growth factor, FGF7), CTGF (connective tissue growth factor) and can be stimulated to release these growth factors. PRP is obtainable from whole blood, which can be both anticoagulated or not anticoagulated. If whole blood or anticoagulated whole blood is centrifugated, it is separated in particular into three different phases:
(i) A first phase comprising the erythrocytes. The first phase is the heaviest phase (having the highest density) and thus the lowest phase of anticoagulated whole blood separated into single phases. The first phase is also called "red phase" or "erythrocytes phase" herein.
(ii) A second phase comprising white blood cells and thrombocytes. The thrombocytes contain the proteins relevant for regeneration (EGF, FGF, IGFs, PDGF, VEGF, TGF-β, IL8, KGF CTGF) in high concentrations. The second phase is lighter than the erythrocytes phase and thus above the erythrocytes phase of anticoagulated whole blood. The second phase is also called "buffy coat" or "PRP" herein.
(iii) A third phase comprising the plasma. The plasma contains proteins, especially albumins, globulins, and clotting proteins including fibrinogen. The plasma is a clear yellowish phase without cells. The third phase is lighter than the first and the second phase and thus the top layer of sedimented and/or centrifugated anticoagulated whole blood. The third phase is also called "plasma" herein.

The term "upright position" of the syringe body (1) as used herein refers to any position of the syringe body (1) in which the syringe opening (22) is positioned below (i.e. below in direction of the gravity force) the syringe opening (23). The upright position may not only comprise a vertical upside-down position (with reference to the gravity force) but also angular positions as long as the syringe opening (22) is positioned below the syringe opening (23). In particular, the term "upright position" as used herein refers to a position of the device or parts thereof as shown in Figs. 1-5.

The term "upside-down position" of the syringe body (1) (or any part of the device as described herein) as used herein refers to any position of the syringe body (1) in which the syringe opening (23) is positioned below (i.e. below in direction of the gravity force) the syringe opening (22). The upside-down position may not only comprise a vertical upside-down position (with reference to the gravity force) but also angular positions as long as the syringe opening (23) is positioned below the syringe opening (22). In particular, the term "upside-down position" as used herein refers to a position of the device or parts thereof which are upside-down in respect of the device or part thereof as shown in Figs. 1-5.

The term "comprising" as used herein shall not be construed as being limited to the meaning "consisting of" (i.e. excluding the presence of additional other matter). Rather, "comprising" implies that optionally additional matter may be present. The term "comprising" encompasses as particularly envisioned embodiments falling within its scope "consisting of" (i.e. excluding the presence of additional other matter) and "comprising but not consisting of" (i.e. requiring the presence of additional other matter), with the former being more preferred.

Fat transfer is a common procedure for the treatment of soft tissue volume deficits. However, the volume loss of the transplant still varies up to 70 %. It is widely accepted that an increased stem cell percentage can increase the take rate of the graft. This can be achieved by a combination of homogenization through mechanical processing using shear forces and separation by centrifugation, resulting in desired phases which can be used for the lipofilling procedure and undesired phases which can be discarded (aspirated blood and tumescence solution or the cytosol and lipids released by disrupted adipocytes during homogenization). The conventional method to achieve this comprises the repeated transfer of desired and undesired phases which is both time and material consuming and bears the risk of contamination with both infectious germs or with the undesired phases. The present invention enables the user to separate the desired phases from the undesired phases with fewer steps and fewer material requirement in less time with decreased risk of contamination with either germs or undesired phases with the latter resulting in a higher yield of a pure product. The present invention simplifies the thorough separation of desired and undesired phases, the discard of the undesired phases, and the application of the desired phase. This is not at all limited to the exemplary processing of lipoaspirate for fat transfer but can be attributed to the clean separation of numerous fluids/substances with the platelet-concentrated plasma phase of separated whole blood being another example.

The present invention refers thus to a device for processing a fluid comprising: a syringe body (1) a syringe plunger (2) comprising a sealing plate (6) and a perforated sealing plate (7) at the lower end of the plunger, and a syringe closure (3), comprising preferably at least one perforation cage (9). The present invention also refers to a syringe plunger (2) and a syringe closure (3) of a device according to the present invention. Moreover, the present invention refers to a method of processing a fluid and a use of the device, the syringe plunger (2), the syringe closure (3) or the method according to the present invention for separating a fluid into two or three phases, for homogenizing and/or enriching the desired cells, for example the stem cells in a fat graft or the platelets in blood.

In a first object of the present invention it is envisaged to provide a device for processing a fluid comprising:
(i) a syringe body (1),
(ii) a syringe plunger (2) comprising a sealing plate (6) and a perforated sealing plate (7) at the lower end of the plunger, and
(iii) a syringe closure (3), comprising at least one perforation cage (9) wherein the device comprises eitherthe perforated sealing plate (7) and at least one perforation cage (9).

More particularly, the present invention refers to a device for processing a fluid by centrifugation and/or sedimentation comprising: (i) a syringe body (1), (ii) a syringe plunger (2) comprising a sealing plate (6) and a perforated sealing plate (7) at the lower end of the plunger, wherein the perforated sealing plate (7) is movably attached to the syringe plunger (2) and the distance between the movable perforated sealing plate (7) and the sealing plate (6) is variable, and (iii) a syringe closure (3), comprising at least one perforation cage (9).

The fluid is preferably a mixture of two or more different substances wherein at least some of the substances differ in their density. The fluid is preferably a fluid mixture and/or a fluid suspension. The fluid mixture is preferably a mixture of liquids. The fluid suspension is preferably a mixture of liquids and solid particles. The fluid may comprise water, lipids, water-soluble substances, liposoluble substances, cells, tissue, proteins, enzymes and/or growth factors. In a preferred embodiment, the fluid comprises cells, in particular mammalian cells. In a highly preferred embodiment of the present invention, the fluid is a sample of a subject such as lipoaspirate, adipose tissue, blood, in particular whole blood or anticoagulated whole blood, a fat graft or bone marrow. The device according to the present invention is in particular for storing and/or separating the fluid into a first, second and optionally third phase. More particularly, it is for the clean and lossless release of individual phases of multiphase fluids after separation using centrifugal or gravitational forces. The processing is preferably performed by sedimentation and/or centrifugation i.e. by applying gravitational or centrifugal force. The separation of the phases is preferably based on differences in density. If the device is kept in an upright position the first phase having the highest density is collected in the syringe closure (3). The second phase having a lower density remains in the syringe body (1) below the sealing plate (6). If the device comprises a perforated sealing plate (7), a third phase having the lowest density is collected in the reservoir between the perforated sealing plate (7) and the sealing plate (6) while the second phase having a higher density than the third phase but a lower density than the first phase remains in the syringe body below the perforated sealing plate. In a highly preferred embodiment the perforated sealing plate (7) is movably attached to the syringe plunger (2) and the distance between the movable perforated sealing plate (7) and the sealing plate (6) is variable. In a preferred embodiment of the present invention this means that the perforated sealing plate (7) is freely movable between the lower end of the plunger and the sealing plate (6). Alternatively, this means that the perforated sealing plate (7) is a releasable (separate) part of the syringe plunger (2) and that the perforated sealing plate (7) is freely movable between the lower end of the syringe body (1) (with respect of the upright position of the device) and the sealing plate (6) as further described below. The perforated sealing plate (7) is preferably designed in that its freely movement results in that the perforated sealing plate (7) aligns between the uppermost phase (the phase with the least density or third phase) and the middle phase (second phase) under the application of gravitational or centrifugal forces.

The term "movably attached" as used herein does not necessarily means that the perforated sealing plate (7) is direclty physically connected with the syringe plunger (2). Said term also comprises any attachment by contact or touch of the perforated sealing plate or a vertical extension thereof with the syringe plunger (2) or parts thereof as its sealing plate (6). In that case, the perforated sealing plate (7) may be disconnected with the part of the syringe plunger (2) comprising the sealing plate (6) as e.g. shown in Fig. 4, Fig. 5E or Fig. 5F. In a preferred embodiment, the term "movably attached" as used herein refers to an attachment of the perforated sealing plate (7) to the part of the syringe plunger (2) comprising the sealing plate (6), wherein its direct attachment may be released. The attachment may be released by simply pulling the sealing plate (6) apart from the perforated sealing plate (7) e.g. by applying a force (15) to the syringe plunger (2). Alternatively, the attachment may be released by a release mechanism. Even if direct attachment of the sealing plate (7) to the part of the syringe plunger (2) comprising the sealing plate (6) is released, the perforated sealing plate (7) is still indirectly attached to the syringe plunger by the guide provided by the syringe body (1).

In a preferred embodiment, the perforated sealing plate (7) is detachable from the part of the syringe plunger (2) comprising the sealing plate (6). In that case the perforated sealing plate (7) is preferably movably attached to the syringe plunger (2) by (i) contact or touch with the sealing plate (6) of the syringe plunger (2), or (ii) a releasable coupling mechanism to the sealing plate (6) of the syringe plunger (2) or the syringe plunger (2). For example, the perforated sealing plate (7) may be attached to the part of the syringe plunger (2) comprising the sealing plate (6) by a rotation mechanism such as a bayonet-catch, wherein rotation of the syringe plunger (2) results in the release or coupling of the perforated sealing plate (7).

If the perforated sealing plate (7) is releasable from the part of the syringe plunger (2) comprising the sealing plate (6), the perforated sealing plate (7) comprises preferably a vertical extension (24) as e.g. shown in Fig. 4. Preferably, said vertical extension (24) extends in a direction vertical to the perforated sealing plate (7) towards the sealing plate (6). Said extension may serve as a distance keeper or separator of the perforated sealing plate (7) and the sealing plate (6). Said vertical extension (24) is designed in that fluid can be collected between the perforated sealing plate (7) and the sealing plate (6). Said vertical extension (24) is preferably also designed in that the sealing function of the perforated sealing plate is maintained. For example, said vertical extension (24) is preferably designed in that it prevents that the perforated sealing plate (7) rotates within the syringe body (1) which would result in a loss of the sealing function of the perforated sealing plate. In a preferred embodiment, the vertical extension has a length (in vertical direction with reference to the perforated sealing plate (7)) of about 0.5 to about 2.5 cm. In a further preferred embodiment, the vertical extension (24) has the same design as the syringe plunger (2) or a common syringe plunger e.g. wall elements vertically arranged (with reference to the perforated sealing plate (7)).

Thus, in a preferred embodiment the syringe plunger (2) (or if it consists of two parts, the lower part of the syringe plunger (2)) comprising the sealing plate (6) and the perforated sealing plate (7) comprises or consists of two parts, namely a first part (lower part (with respect of the upright position of the device)) comprising the perforated sealing plate (7) and its vertical extension (24) and the second part (upper part (with respect of the upright position of the device)) comprising the sealing plate (6).

In a preferred embodiment, the device of the present invention is a syringe system for processing lipoaspirate. It is in particular for separating lipoaspirate into a first, second and third phase. In particular, the syringe system provided by the present invention is for separating lipoaspirate into three phases, namely
(i) a tumescent phase (first phase),
(ii) a fat graft phase (second phase), and
(iii) an oily phase (third phase).

The tumescent phase is collected in the syringe closure (3) and may be discarded after centrifugation. The oily phase is collected in the reservoir between the perforated sealing plate (7) and the sealing plate (6). The fat graft phase remains in the syringe body (1) below the perforated sealing plate (7) and is directly ready for further use.

In another embodiment of the present invention, the device is preferably a syringe system for processing blood, in particular whole blood or anticoagulated whole blood. It is in particular for storing and/or separating blood into a first, second and third phase. In particular, the syringe system provided by the present invention is for separating blood into three phases, namely
(i) erythrocytes phase (first phase),
(ii) PRP (second phase), and
(iii) plasma (third phase).

For processing fluids such as blood, the device according to the present invention is preferably kept or centrifugated in an upside-down position. Accordingly, the first phase having the highest density as e.g. the erythrocyte phase is collected in the reservoir between the perforated sealing plate (7) and the sealing plate (6). The plasma is collected in the syringe closure (3). For centrifuging the device in the upside-down position, the syringe plunger is preferably cut about 1 to about 5 cm, about 2 to about 4 cm, about 1 to about 3 cm or about 3 to about 5 cm above the finger flange (4) (after the device has been filled with fluid). This results in that the centrifugal force pushes the syringe plunger inside the syringe body which may lead to a compression of the air in the syringe closure (3). To improve the outcome the compressed air may be ejected by a pressure relief valve, a membrane, preferably a hydrophobic membrane, during the centrifugation.

Said pressure relief valve or membrane is preferably located in the syringe closure (3). The upside-down position is preferably obtained after the device according to the present invention has been filled with a fluid for further processing.

Alternatively, if the device is used for processing blood and the device is kept in its upright position, the erythrocyte phase is collected in the syringe closure (3) and may be discarded after centrifugation. The plasma is collected in the reservoir between the perforated sealing plate (7) and the sealing plate (6).

In both embodiments, the highly concentrated PRP remains in the syringe body (1) between the perforated sealing plate (7) and the syringe opening (22). The highly concentrated PRP is directly ready for further use. For example, after separation from the first phase the syringe comprising the second phase may be connected to e.g. another syringe containing for example cell enriched lipoaspirate e.g. obtained by using a device according to the present invention or obtained by performing the method according to the present invention which can then be combined with the highly concentrated PRP.

In another embc diment, the syringe system is for storing and/or separating blood into a first and a second phase, namely
(i) erythrocytes phase (first phase), and
(ii) second phase comprising the buffy coat and the plasma (high volume PRP).

This embodiment is in particular useful when a higher volume (and lower platelet concentration) is desired. Preferably, the device is kept or centrifugated again upside-down as described above. In this embodiment, the device according to the present invention may comprise a syringe closure (3) of minimal volume (preferably of about 0 to about 7 ml, more preferably of about 0 to about 5 ml) without any perforation cage (9). Accordingly, the erythrocyte phase is collected between the sealing plates (6 and 7).

Alternatively, if the device is used for processing blood and the device is kept in its upright position, a common syringe plunger having only a sealing plate (6) but no perforated sealing plate (7) may be used. In this embodiment, the erythrocyte phase is collected in the syringe closure (3) and may be discarded after centrifugation.

In both embodiments, highly volume PRP remains in the syringe body (1) between the perforated sealing plate (7) (or the sealing plate (6) if there is no perforated sealing plate (7)) and the syringe opening (22). The syringe comprising the second phase may be connected to another syringe containing for example cell enriched lipoaspirate e.g. obtained by using a device according to the present invention or obtained by performing the method according to the present invention which can then be combined with the high volume PRP.

The separation of the fluid (lipoaspirate) may be performed by sedimentation or centrifugation or by sedimentation and centrifugation. The second phase obtained from said separation remains in the syringe body and is immediately ready for further use as e.g. storage, transplantation, or lipofilling. The aim of said separation is preferably the provision of the second phase comprising vital adipocytes, progenitor cells of the stromal vascular fraction (SVF), adipose stem cells and growth factors which can be used for lipofilling, in particular for the treatment of age-, disease- or trauma-related soft tissue volume deficits. Accordingly, the device or syringe system according to the present invention is preferably configured to be operable as a closed sterile device for avoiding any contamination of the subject. In particular, the device or syringe system and its single components are configured to be sterilizable.

The syringe body has preferably the form of a cylinder having a syringe opening (22) at its lower end and a syringe opening (23) at its upper end. The syringe opening (23) forms the opening of the syringe body (1) for receiving the syringe plunger (2) while the syringe opening (22) forms the opening which is usually used as inlet and outlet opening for receiving and discarding any fluid. Accordingly, syringe opening (22) has preferably a smaller diameter than syringe opening (23). In a preferred embodiment syringe opening (22) is cone-shaped. Preferably, the syringe body comprises at syringe opening (22) a luer-lock connection (5), more preferably a male luer-lock connection. The male luer-lock connection is preferably configurated to receive a liposuction cannula, a lipofilling cannula, a syringe needle, a connector, or a three-way stop cock. In addition, the syringe body (1) comprises at syringe opening (23) a finger flange (4). The syringe body is preferably made from glass, plastic or medical steel.

The syringe plunger (2) is preferably configured to be inserted into the syringe body (1) through syringe opening (23) and to be movable within the syringe body (1). It has preferably a finger flange at its upper end. The sealing plate (6) is preferably arranged at the lower end of the syringe plunger (2). The optional perforated sealing plate (7) is preferably arranged at the lower end of the syringe plunger and the sealing plate (6) is arranged above the perforated sealing plate (7) and below the finger flange i.e. in between the finger flange and the perforated sealing plate (7). The perforated sealing plate (7) is preferably attached to the lower part of the syringe plunger or it is positioned below the lower part of the syringe plunger. Preferably, the perforated sealing plate (7) forms the lower end of the syringe plunger (2). The terms "lower end" and "upper end" refers to an upright position of the syringe plunger which is receivable by the syringe body (1) in its upright position. The perforated sealing plate (7) may be fixed at the lower part of the syringe plunger or it may be movably attached to the syringe plunger (2) as described in detail above. In a highly preferred embodiment of the present invention, the perforated sealing plate (7) is movably attached to the syringe plunger (2). If the perforated sealing plate (7) is movably attached to the syringe plunger (2), the perforated sealing plate (7) can be pushed or pulled up and down along the plunger or the syringe body for forming a reservoir having a variable volume for receiving a liquid phase or gas between the perforated sealing plate (7) and the sealing plate (6). This preferably means that the attachment of the perforated sealing plate (7) with the syringe plunger (2) is designed so that the perforated sealing plate (7) is freely movable between the lower end of the plunger and the sealing plate (6). Preferably, the term "freely movable" does not refer to any rotating movement by which the sealing function of the perforated sealing plate is lost. In contrast, the term "freely movable" refers preferably to a movement of lifting and lowering the perforated sealing plate (7) along the plunger or along the syringe body (1). Thus, even if the perforated sealing plate (7) is freely movable between the lower end of the plunger and the sealing plate, it keeps its sealing function so that any fluid flow through the perforated sealing plate is limited to any fluid flow through its perforations. Thus, the perforated sealing plate (7) is preferably designed in that it is able to move along the plunger or along the syringe body without losing its sealing function. More particularly, the attachment of the perforated sealing plate (7) with the syringe plunger (2) is designed so that the perforated sealing plate aligns between the uppermost phase (the phase with the least density or third phase) and the middle phase (second phase) under the application of gravitational or centrifugal force. In a preferred embodiment, the density of the movable perforated sealing plate (7) is lower than the density of the phase beneath it, so that it is swimming on top of the phase beneath it. Thus, the perforated sealing plate is preferably designed to have a density being lower than the density of the second phase (middle phase) but higher than the density of the first phase (uppermost phase). In a further preferred embodiment, the density of the movable perforated sealing plate is about 1.05 to about 1.09 g/ml, in particular for treatments, like the upside-down centrifugation of blood. In a further preferred embodiment, the density of the movable perforated sealing plate is about 0,85 to about 0,89 g/ml, in particular for the separation of lipoaspirate in an upright position.

If the perforated sealing plate (7) is fixed at the lower part of the syringe plunger (2), the perforated sealing plate (7) and the sealing plate (6) are configured to form a reservoir having a fixed volume for receiving a liquid phase or gas between the perforated sealing plate (7) and the sealing plate (6). Preferably, the sealing plate (6) is arranged in the lower half or in the lower third of the syringe plunger (2). The sealing plate (6) is preferably fixed to the syringe plunger (2) in a position above the perforated sealing plate (7). To fix in different centrifuge-cups the finger flange is preferably removeable with a bayonet-catch or by cutting.

The embodiment of having a variable volume for receiving in particular a liquid phase between the perforated sealing plate (7) and the sealing plate (6) is particularly preferred if the device is kept or centrifugated upside down as described above but it is also preferred if the device is kept or centrifugated in its upright position. The embodiment of having a variable volume between the perforated sealing plate (7) and the sealing plate (6) is the most preferred embodiment of the present invention.

Thus, according to one embodiment, the syringe plunger (2) comprises a sealing plate (6) and a perforated sealing plate (7) at the lower end of the plunger, wherein the sealing plate (6) is fixed to the syringe plunger (2) and the distance between the perforated sealing plate (7) and the sealing plate (6) is constant. According to the present invention, the syringe plunger (2) comprises a sealing plate (6) and a perforated sealing plate (7) at the lower end of the plunger, wherein the perforated sealing plate (7) is movably attached to the syringe plunger (2) and the distance between the perforated sealing plate (7) and the sealing plate (6) is variable.

In both embodiments of having a fixed volume reservoir or a variable volume reservoir as described above, the syringe plunger (2) may consists of one or at least two parts. In a preferred embodiment, the syringe plunger (2) comprises at least two parts, namely a lower part and an upper part. The lower part comprises the perforated sealing plate (7) and the sealing plate (6). The upper part may be configured to be removable from the syringe body, wherein the sealing plate (6) remains in the syringe body (1) and keeps sealing the fluid in the syringe body (1) after the upper part of the syringe plunger (2) has been removed. In a preferred embodiment, the sealing plate (6) or the lower part of the syringe plunger (2) is removably attached to the upper part of the syringe plunger (2), i.e. it can be removed from the upper part of the syringe plunger (2). For example, the upper part of the syringe plunger may be configured to be removed from the lower part of the syringe plunger by separating the upper part from the lower part e.g. by cutting the upper part off. In a preferred embodiment of the present invention, the upper part of the syringe plunger is removably attached to the lower part of the syringe plunger or the sealing plate (6), i.e. it is preferably configured to be removable from the lower part of the syringe plunger or the sealing plate (6) and is configured to be attachable to the lower part of the syringe plunger or the sealing plate (6) after it has been removed. Preferably, the upper and lower part of the syringe plunger and the sealing plate, respectively, are removably attached by a bayonet-catch. The advantage of using a syringe plunger (2) which is completely or partly removable is that after removing the plunger or a part thereof the syringe system fits into common centrifuges. Thus, in a preferred embodiment of the present invention, the syringe plunger comprises a lower part comprising the sealing plate (6) and the perforated sealing plate (7) and an upper part, wherein the upper part of the syringe plunger is removable from the lower part of the syringe plunger e.g. by a bayonet-catch. Preferably the upper part of the syringe plunger is removed or cut about 1 to about 5 cm, about 2 to about 4 cm, about 1 to about 3 cm or about 3 to about 5 cm above the finger flange (4) (after the device has been filled with fluid).

If the perforated sealing plate (7) is freely movable as described above, the syringe plunger (2) (or if it consists of two parts, the lower part of the syringe plunger (2)) comprising the sealing plate (6) and the perforated sealing plate (7) may comprise or consists or two parts as described above, namely a first part (lower part (with respect of the upright position of the device)) comprising the perforated sealing plate (7) and its vertical extension (24) and the second part (upper part (with respect of the upright position of the device)) comprising the sealing plate (6).

Thus, in a preferred embodiment, the syringe plunger (2) may consists of three parts, namely (a) an upper part being removable so that the device fits into common centrifuges as described above, (b) a lower part comprising the sealing plate (6) and (c) a third (lowest) separate or releasable part as described above, wherein said third part comprises the perforated sealing plate (7) and its vertical extension (24).

Preferably, the sealing plate (6) is configured to move with the syringe plunger within the syringe body. The sealing plate (6) is preferably configured to seal the environment above the sealing plate (6) from the environment below the sealing plate, in particular for fluids, liquids and gas as e.g. ambient air.

In a preferred embodiment of the present invention, the perforated sealing plate (7) is fixed to the syringe plunger (2). Preferably, the perforated sealing plate (7) is configured to move with the syringe plunger within the syringe body.

The perforated sealing plate (7) is preferably configured to seal the environment above the perforated sealing plate (7) from the environment below the sealing plate, in particular for fluids, liquids and gas as e.g. ambient air at its outer perimeter. The openings of the perforations are preferably configured to be permeable for gas and liquids, in particular for gas and the oily phase of a lipoaspirate or plasma of blood but not permeable for the components of the second phase due to its tissue structure. The perforated sealing plate (7) comprises at least 2 perforations or openings (10), more preferably about 2 to 16 perforations or openings. Said openings are preferably circular arranged in the perforated sealing plate (7).

The openings (10) have preferably a size of about 0.5 µm to about 0.8 mm. In a preferred embodiment, the openings (10) are circular and have a diameter of about 0.2 µm to about 0.8 mm. However, the opening may also be oval, triangular, rectangular, square or multi-sided and have a perimeter of about 0.6 µm to about 2.5 mm.

If the device is particularly designed for processing lipoaspirate or adipose tissue, the openings have preferably a diameter of about 0.2 mm to about 0.8 mm or a perimeter of about 0.6 mm to about 2.5 mm, or more preferably of about 0.05 mm to about 0.5 mm or a perimeter of about 0.2 mm to about 1.6 mm. If the device is particularly designed for processing blood, the openings have preferably a diameter of about 0.2 µm to about 2.5 µm or a perimeter of about 0.6 µm to about 7.9 µm.

The perforated sealing plate (7) forms preferably the lower end of the syringe plunger (2). The sealing plate (6) is arranged above the perforated sealing plate (7), preferably in a distance of about 1 to about 5 cm, more preferably of about 2 to about 4 cm, for moulding a reservoir to storage liquid and/or gas as e.g. the oily phase, plasma and/or ambient air. In a preferred embodiment of the present invention, the volume for gas and liquid between the sealing plate (6) and the perforated sealing plate (7) is a fixed volume of about 1 to about 18 ml or it is a variable volume in the range of about 0 to about 18 ml, if the syringe plunger (2) is inserted into the syringe body. Thus, in a preferred embodiment of the present invention, the sealing plate (6) and the perforated sealing plate (7) form a reservoir of about 1 to about 18 ml between them for storing liquid and/or gas as e.g. an oily phase, plasma and/or ambient air.

The syringe plunger is preferably made from glass, plastic or medical steel.

The syringe closure (3) is preferably a container having an opening at its top and upper end, respectively. The top of the syringe closure (3) is preferably removably connectable to the lower end of the syringe body, i.e. the end of the syringe body comprising syringe opening (22). Said connection is preferably a luer-lock connection. Preferably the syringe body (1) comprises a male luer-lock connection and the syringe closure comprises a female luer-lock connection.

The bottom of the syringe closure (3) may be straight or conical. It is preferably straight if separation is performed by sedimentation. If the separation is performed by centrifugation, the bottom of the syringe closure (3) is preferably conical for fitting into common centrifuges.

The syringe closure (3) comprises an opening (8) for receiving liquids from the syringe body (1). Said opening (8) comprises preferably a female luer-lock connection. In addition, the syringe closure (3) comprises a collection cup (21) for collecting the first phase. In a preferred embodiment, the syringe closure comprises at least one perforation cage (9) between the opening (8) and the collection cup (21). The perforation cage (9) is configured in that any liquid passing from the opening (8) to the collection cup (21) has to pass at least one perforation cage (9). The ring (18) stabilises the connected syringe closure (3) and the syringe body (2) during pressure (16), like during centrifugation, and prevents a mechanical load of the luer-connection.

If the syringe closure (3) comprises more than one perforation cage, each further perforation cage is configured in that any liquid passing from the first or previous perforation cage has to pass the second or subsequent perforation cage for reaching the collection cup. Thus, if the syringe system comprises for example four perforation cages, any fluid passing the opening (8) reaches only the collection cup (21) after passing first the first perforation cage, subsequently the second perforation cage, subsequently the third perforation cage and at last the fourth perforation cage prior for reaching the collection cup.

The perforation cages are configured to filter the fluid, liquid or suspension. In particular, the perforation cages are configured in that the heavier first phase (12) passes through the cage openings (19.1, 19.2) while the second phase is prevented from passing the perforation cages due to its tissue-structure components. Moreover, as described above, some substances of the fluid such as collagen fibres may seal the openings of the perforation cages so that nearly no volume loss of the second phase is observed.

In one embodiment of the present invention, the perforation cage may be formed by a net, membrane or linear perforation plate which is placed between the opening (8) to the collection cup (21). The perforation plate may be designed similar or like the perforated sealing plate (7) as described above. This embodiment is particularly suitable if the syringe system according to the present invention is for non-centrifugation use, wherein the separation of the three phases is performed by sedimentation.

In an especially preferred embodiment of the present invention, the perforation cage(s) has/have preferably the form of a cylinder with a bottom at its lower end (with respect of the upright position of the syringe closure). The upper end of the perforation cage is open for receiving liquid from the syringe body (1). Preferably the opening of the perforation cage forms an extension of the female luer-lock connection. If the syringe closure (3) comprises more than one perforation cage, the upper end of the second and each further perforation cage is open for receiving liquid from the previous perforation cage. The diameter of the opening of the perforation cage of the second and each further perforation cage may be smaller than the diameter of the previous perforation cage, i.e. the dimension of a subsequent perforation cage is preferably bigger than the dimension of the previous perforation cage. The bottom of the perforation cage has at least two vertical cage openings (19.2) more preferably about 2 to about 16 vertical cage openings (19.2). The vertical cage openings are preferably circular arranged on the bottom of the perforation cage. The vertical cage openings are preferably circular, preferably with a diameter of about 0.2 µm to about 0.8 mm. However, they may also be oval triangular, rectangular, square or multi-sided and have a perimeter of about 0.6 µm to about 2.5 mm. The bottom of the perforation cage may be curved or straight. In a preferred embodiment it is straight.

If the device is particularly designed for processing lipoaspirate or adipose tissue, the vertical cage openings have preferably a diameter of about 0.2 mm to about 0.8 mm or a perimeter of about 0.6 mm to about 2.5 mm, or more preferably of about 0.05 mm to about 0.5 mm or a perimeter of about 0.2 mm to about 1.6 mm. If the device is particularly designed for processing blood, the openings have preferably a diameter of about 0.2 µm to about 2.5 µm or a perimeter of about 0.6 µm to about 7.9 µm. The sizes of the openings may be adapted to the needs of further fluids and the phases into which they are to be separated by the device according to the present invention.

In a preferred embodiment of the present invention, the perforation cage(s) comprise(s) additionally at least one horizontal cage opening (19.1), more preferably about 1 to 12 horizontal cage openings (19.1). The horizontal cage openings (19.1) are preferably circular and have preferably a diameter of about 0.2 µm to about 0.8 mm. However, they may also be oval, triangular, rectangular, square or multi-sided and have preferably a perimeter of about 0.6 mm to about 2.5 mm or more preferably of about 0.6 µm to about 2.5 mm. Preferably, the diameter of the horizontal cage openings (19.1) is the same as the diameter of the vertical cage openings (19.2) of the respective perforation cage. The horizontal cage openings (19.1) are preferably arranged at the side wall of the cylinder. Preferably, they are circular arranged at the side wall of the cylinder.

If the device is particularly designed for processing lipoaspirate or adipose tissue, the horizontal cage openings have preferably a diameter of about 0.2 mm to about 0.8 mm or a perimeter of about 0.6 mm to about 2.5 mm, or more preferably of about 0.05 mm to about 0.5 mm or a perimeter of about 0.2 mm to about 1.6 mm. If the device is particularly designed for processing blood, the openings have preferably a diameter of about 0.2 µm to about 2.5 µm or a perimeter of about 0.6 µm to about 7.9 µm. The size of the openings may be adapted to the needs of further fluids and the phases into which they are to be separated by the device according to the present invention.

In a preferred embodiment of the syringe system according to the present invention, the syringe closure comprises 2, 3, or 4 perforation cages.

If the syringe closure comprises more than one perforation cage, the size of the opening of each subsequent perforation cage is preferably equal, more preferably below the size (i.e. diameter or perimeter) of the opening of the previous perforation cage. For example, if the openings (19.2 and optionally 19.1) of the first perforation cage (i.e. the perforation cage receiving any liquid from the syringe body first) have a diameter of 0.8 mm, then the openings (19.2 and optionally 19.1) of the second perforation cage (i.e. the perforation cage receiving any liquid from the first perforation cage (20.2 and optionally 20.1)) may have a diameter of 0.2 mm.

The syringe closure (3) is preferably made from glass, plastic or medical steel.

The device, in particular the syringe closure (3), is designed for withstanding gravitational force (preferably about 0.9 to about 1.1 g, more preferably about 1 g (normal acceleration of gravity) or centrifugal force such as of about 1 to about 5000 rcf, more preferably of about 100 to about 4000 rcf, more preferably of about 400 to about 3000 rcf. It is preferably designed to prevent a mechanical load of the luer-connection during centrifugation. For example, the syringe closure is designed to fit into common centrifuges. Therefore, the syringe closure (3) has preferably a round cross section having a diameter of about 28 mm to 32 mm, more preferably of about 30 mm. The gravitational force e.g. for sedimentation is preferably of 0.9 to about 1.1 g, more preferably of about 1 g (normal acceleration of gravity). The centrifugal force for e.g. centrifugation is preferably of about 1 rcf to about 5000 rcf, of about 100 rcf to about 5000 rcf, or of about 400 to 3000 rcf for centrifugation.

In a preferred embodiment of the present invention, the syringe closure (3) additionally comprises a ring (18) which is configured to contact both the syringe body (1) and the syringe closure (3). In particular, the ring (18) is configured to cover the connection, as e.g. the luer-lock connection, between the syringe body (1) and the syringe closure (3). Thus, ring (18) is preferably a force absorption ring for protecting the luer-lock connection between the syringe body (1) and the syringe closure (3) during centrifugation. Ring (18) is preferably made from glass, plastic or steel.

The present invention also refers to a syringe plunger (2) of a device or syringe system of the present invention comprising a sealing plate (6) and a perforated sealing plate (7) at the lower end of the plunger. Said syringe plunger (2) has preferably the same features as the syringe plunger (2) described herein. In particular, the syringe plunger comprises preferably a lower part comprising the sealing plate (6) and the perforated sealing plate (7) and an upper part, wherein the upper part of the syringe plunger is removable from the lower part of the syringe plunger as described in further details above. In an especially preferred embodiment, the perforated sealing plate (7) is movably attached to the syringe plunger (2) and the distance between the perforated sealing plate (7) and the sealing plate (6) is variable as further described herein. Thus, the syringe plunger (2) may also comprise or consist of two or three parts, wherein one part is formed by the perforated sealing plate (7) and its vertical extension (24) as e.g. shown in Fig. 4.

The present invention also refers to a syringe closure (3) of a syringe system of the present invention comprising at least one perforation cage (9). Said syringe closure (3) has preferably the same features as the syringe closure (3) as described above.

In a preferred embodiment, the device or syringe system according to the present invention is preferably configured to be operable as follows: Like common syringe systems, the syringe system according to the present invention can be filled with a liquid or suspension like lipoaspirate (11) by inserting the syringe plunger into the syringe body and moving the syringe plunger (2) upwards by a force (15). After filling the syringe system, the syringe closure (3) will be connected to the syringe body (1) and the syringe plunger (2) may be shortened e.g. by using a bayonet-closure above the sealing plate (6). Subsequently, the constituents will be separated by sedimentation or centrifugation. If a force (16) is applied to syringe plunger (2) the sedimented oily phase of the lipoaspirate (14) passes through the perforated sealing plate (7). Due to the tissue-structure, the fat phase is not able to pass the openings and remains inside the reservoir between the sealing plate (6) and the perforated sealing plate (7) and therefore inside the syringe body (1). Furthermore, the collagen-fibres of the fat tissue preferably seal the openings after force (16) have been applied to prevent the oily phase (14) to flow back inside the syringe body (1). The sealing plate (6) does not have any openings. Hence, the stored oily phase (14) remains in the reservoir between the sealing plate (6) and the perforated sealing plate (7). Depending on the dimensioning, preferably 1 to 18 ml can be stored in this reservoir.

After separation of the three different phases by sedimentation or centrifugation, the tumescent phase in the collection cup of the syringe closure can be rejected. The sterile processed fat below the perforated sealing plate (7) can be released by applying a force (17) on the syringe plunger. Usually force (17) is applied by pressing the syringe plunger manually.

For the recommended CELT procedure, the once centrifuged fat (1-5 minutes with 400-3000 rcf), should be homogenized. Therefore, the syringe closure is removed and a luer-lock to luer-lock connector 30 to 180 degrees with an inner diameter of 0.6 to 2.8 mm, 0.2 mm to 2.0 mm or 0.2 to 2.8 mm is connected. Said inner diameter is preferably variable, which means that the size can be reduced. For examples, the reduction of the inner diameter can be achieved by closing the connector partially e.g. by using a cock or tab Another syringe body (1) including a syringe plunger (2) is added at the other side of the connector. The homogenization comprises pressing the fat from one syringe to the other manually 3 to 21 times. Due to shear forces during intersyringe processing the size of e.g. the large-scaled adipose tissue particles decreases. Said decrease is preferably caused in view of the reduction of the inner diameter of the connector i.e. as the connector has a smaller inner diameter than the syringe inlet and outlet. Afterwards, the homogenized fat should remain in the new syringe. After shortening the syringe plunger and adding a new syringe closure (3), the syringe can be centrifuged again at 400-3000 rcf for 1-5 minutes. Homogenization disrupts the majority of mature adipocytes, resulting in released lipids and cytosol. As during the first centrifugation, the oily, fat and tumescent phases are separated and stored in the reservoir between the sealing plates (6 and 7) and inside the syringe closure (3). The remaining fat-phase can be directly injected into the patient, using a lipofilling cannula or homogenised again and applied like a filler. The autologous filler can also be stored for a later application Alternatively, the remaining fat-phase can be mixed with PRP to increase the growth factor concentration and hence the take rate or with once centrifuged fat to increase the volume of the fat graft. The homogenization and centrifugation can be repeated 1 to 4 times to increase the cell concentration of (Adipose-derived Mesenchymal Stem/Stromal Cells (AD-MSCs), Hematopoietic stem cells (HSCs), Regulatory T cells (T_{reg} Cells), Adipocytes, Pericytes, Endothelial Cells (ECs), mast-cells, fibroblasts, dendritic cells, intra-adventitial smooth muscular-like cells), the extracellular matrix and growth factors.

Thus, the present invention also provides a method of processing a fluid, the method comprises the steps of:
(a) Providing a syringe body (1) filled with a fluid (11), wherein the plunger comprises a sealing plate (6) and optionally a perforated sealing plate (7) at its lower end;
(b) Connecting a syringe closure (3) to the syringe body (1), wherein the syringe closure (3) comprises at least one perforation cage (9); and
(c) Separating the fluid into two or three phases by centrifugation or sedimentation,
wherein the device comprises either the perforated sealing plate (7) or at least one perforation cage (9) or both.

In a particularly preferred embodiment, the present invention provides a method of processing a fluid, the method comprises the steps of:(a) Providing a syringe body (1) filled with a fluid (11), wherein the plunger comprises a sealing plate (6) and a perforated sealing plate (7) at its lower end, wherein the perforated sealing plate (7) is movably attached to the syringe plunger (2) and the distance between the movable perforated sealing plate (7) and the sealing plate (6) is variable; (b) Connecting a syringe closure (3) to the syringe body (1), wherein the syringe closure (3) comprises at least one perforation cage (9); and (c) Separating the fluid into two, three or more phases by centrifugation or sedimentation.

The method of processing the fluid is preferably a method of separating the fluid into two or three phases. Preferably, it is also a method for the clean and lossless release of individual phases of multiphase fluids after separation using centrifugal or gravitational forces. The fluid is as defined above and is, in a preferred embodiment, a sample of a subject. Providing a syringe body (1) filled with a sample (11) of a subject does not comprise obtaining the sample (11) of a subject. Rather, it means that a syringe body (1) comprising a sample (11) of a subject is provided after the sample has been obtained from the subject. It is possible to obtain the sample of the subject by using the syringe body (1) and the syringe plunger (2) of the device according to the present invention, in particular by applying a tractive force (15) to the plunger of a syringe (2) but the step of obtaining the sample is envisaged not to be a step of the method according to the present invention.

Step c) is preferably performed by centrifugation. Preferably centrifugation is performed once. Preferably centrifugation is performed for about 1 to about 5 minutes at about 400 to about 3000 rcf.

The fluid which is processed by this method is preferably a mixture of two, three or more phases having different densities.

The method of processing a fluid of a subject is preferably a method of processing lipoaspirate or adipose tissue or a method of processing blood, such as whole blood more preferably anticoagulated whole blood into a first, second and optional third phase as described above.

In a first embodiment of the present invention, the fluid is processed in an upright position of the device according to the present invention into a first phase (having a higher density than the second and third phase, a second phase (having a higher density than the third phase) and a third phase , wherein the first phase reaches the syringe closure (3), the second phase remains in the syringe body (1) below the perforated sealing plate (7) and the third phase remains in the syringe body (1) between the perforated sealing plate (7) and the sealing plate (6). The perforated sealing plate (7) of this embodiment is preferably movably attached to the syringe plunger (2) as further described herein. Alternatively, if the syringe plunger (2) does not comprise a perforated sealing plate (7), the first phase reaches the syringe closure (3) and the second phase remains in the syringe body (1) between the sealing plate (6) and syringe opening (22).

In a second preferred embodiment of the present invention, the fluid is processed in an upside-down position of the device according to the present invention into a first phase (having a higher density than the second and third phase), a second phase (having a higher density than the third phase) and a third phase, wherein the third phase reaches the syringe closure (3), the second phase remains in the syringe body (1) between syringe opening (22) and the perforated sealing plate (7) and the first phase remains in the syringe body (1) between the perforated sealing plate (7) and the sealing plate (6). This embodiment is preferably performed for processing blood, whole blood or anticoagulated whole blood into highly concentrated PRP. Alternatively, if the fluid is processed in an upside-down position only into a first phase (having a higher density than the second phase) and a second phase, no phase reaches the syringe closure (3), the second phase remains in the syringe body (1) between syringe opening (22) and the perforated sealing plate (7) and the first phase remains in the syringe body (1) between the perforated sealing plate (7) and the sealing plate (6). This embodiment is preferably performed for processing blood, whole blood or anticoagulated whole blood into high volume PRP. The perforated sealing plate (7) of this embodiment is preferably movably attached to the syringe plunger (2) as further described herein.

The device according to the present invention can also be used for performing a Cell Enriched Lipo-Transfer (CELT). By CELT the fat graft lobule size is minimized and cells, in particular progenitor cells and/or stem cells, are enriched. The resulting micro- or nanofat is particularly useful if transplantation must be performed with small sized cannulas, like face lipofilling. Furthermore, micro- or nanofat may increase the take rate of the transplant since it is enriched in progenitor cells. In particular, the stem cells in the fat phase may be enriched by CELT. CELT is preferably a method of homogenizing the fat phase.

For performing CELT, the method according to the present invention comprises additionally the steps of:
(i) Connecting the syringe body (1) to a container by a connector, preferably by a luer to luer connector; and
(ii) Pushing or pulling the fluid from the syringe body (1) through the connector into the container; and optionally
(iii) Pushing or pulling the fluid back into the syringe body (1).

Steps (ii) and (iii) are preferably performed by applying a force to the syringe plunger (2). Preferably, the fluid is pushed through the connector into the container by pushing the syringe plunger (2) towards the lower end of the syringe body (1), i.e. by applying a compressive force onto the syringe plunger (2).

The connector has preferably a small inner diameter like as e.g. about 0.6 mm to about 2.8 mm, more preferably about 1 mm to about 2 mm, or about 1.1 mm to about 1.5 mm, or of about 1.2 mm to about 1.4 mm. Preferably, the connector is a luer-lock to luer-lock connector 30 to 180 degrees. In a preferred embodiment of the present invention, the connector is a 90 degrees luer-lock to luer-lock connector with an inner diameter of 0.6 to 2.8 mm, 0.2 mm to 2.0 mm, 0.2 to 2.8 mm or about 1.2 mm. Said inner diameter is preferably variable, which means that the size can be reduced. For examples, the reduction of the inner diameter can be achieved by closing the connector partially e.g. by using a cock or tab.

The container and the connector can be combined in one device as long as it comprises a narrow section having an inner diameter of about 0.6 mm to about 2.8 mm, more preferably of about 1 mm to about 2mm, or of about 1.1 mm to about 1.5 mm, or of about 1.2 mm to about 1.4 mm through which the fluid is pressed or pulled before it reaches a repository or container. In a preferred embodiment of the present invention, the container is a second syringe. Preferably said syringe comprises a syringe plunger (2) of the present invention having a sealing plate (6) and a perforated sealing plate (7). The syringe body (1) of the second syringe is preferably connectable to a syringe closure (3) of the present invention. If the container is a second syringe, the fluid may also be pulled through the connector into the second syringe by pulling the syringe plunger (2) of the second syringe towards the upper end of the syringe body (1) of the second syringe i.e. by applying a tractive force onto the syringe plunger (2) of the second syringe. For achieving higher shear forces, the fluid is preferably pushed through the connector back and forth. Thus, step (ii) is preferably performed by applying a compressive force to the plunger of the first syringe (2) but it may also be performed by applying a tractive force to the syringe plunger of the second syringe. Step (iii) is preferably performed by applying a compressive force to the plunger of the second syringe but it may also be performed by applying a tractive force to the syringe plunger (2) of the first syringe.

Preferably, steps ii) and iii) are repeated several times. Preferably step ii) is repeated 1 to 10 times and step iii) is repeated 0 to 9 times. Preferably, the fluid is pushed or pulled three, four, five, six, seven, eight, nine, ten, eleven, twelve, thirteen, fourteen, fifteen, sixteen, seventeen, eighteen, nineteen, twenty, twenty-one or more times through the connector. Preferably, the pushing or pulling is stopped if the fluid is in the container, preferably in the second syringe. This has the advantage that the optional third phase can be easily obtained from the first syringe. In an especially preferred embodiment, the fluid is pushed or pulled eleven times through the connector, i.e. step ii) is repeated five times and step ii) is repeated four times.

The method according to the present invention may comprise an additional step (d) of removing the syringe closure (3) from the syringe body (1) and/or an additional step (iv) of removing the container and the connector from the syringe body (1). Moreover, the method according to the present invention may additional comprise a step of pre-processing the fluid before the fluid is separated into distinct phases and thus prior to step (c). For example, such a pre-processing may comprise a collagen digestion e.g. by the addition of active compounds such as enzymes.

Steps (i) to (iii) may be performed subsequent to step (a) and prior to step (b). However, for obtaining a higher quality of the phases, in particular of the second phase, steps (i) to (iii) are preferably performed subsequent to step c).

The fluid for which CELT is performed and which is pushed or pulled through the connector is preferably the fluid provided in step a) if steps (i) to (iii) are performed subsequent to step (a) and prior to step (b). Preferably said fluid is lipoaspirate or adipose tissue. If steps (i) to (iii) are performed subsequent to step (c), the fluid which is pushed or pulled through the connector is preferably the second phase obtained in step (c).

In steps (ii) and (iii) preferably the size of large-scaled adipose tissue particles decreases due to shear forces during intersyringe processing. This shear force also disrupts mature adipocytes, releasing their lipids and cytosol. Hence, after performing the CELT method steps (b) and (c) of the method according to the present invention is preferably performed. Preferably, repeated step (c), (called c' in the following) is performed by centrifugation, for example for about 1 to about 5 min at about 400 to about 3000 rcf, more preferably for about 1 to about 3 min at about 1400 to about 1800 rcf, more preferably for about 2 min at about 1600 rcf. Said separation results in particular in the separation of the lipids from the concentrated fat graft. In particular, the oily phase from the disrupted adipocytes is pushed due to centrifugal force to the reservoir between the sealing plate (6) and the perforated sealing plate (7) and the cytosol phase is pushed through the perforation cages (9) to the syringe closure (3). The remaining fat-phase in the syringe body (1) below the perforated sealing plate (7) is enriched in stem cells and is immediately ready for further use as e.g. injection into the patient using a lipofilling cannula.

Alternatively, the remaining fat-phase can be mixed with PRP to increase the growth factor concentration and hence the take rate or with once centrifuged fat to increase the volume of the fat graft. The homogenization and centrifugation can be repeated 1 to 4 times to increase the cell concentration of (Adipose-derived Mesenchymal Stem/Stromal Cells (AD-MSCs), Hematopoietic stem cells (HSCs), Regulatory T cells (T_{reg} Cells), Adipocytes, Pericytes, Endothelial Cells (ECs), mast-cells, fibroblasts, dendritic cells, intra-adventitial smooth muscular-like cells), the extracellular matrix and growth factors.

If steps (i) to (iii) are performed subsequent to step (c), steps (b) and (c) are preferably repeated subsequent to steps (i) to (iii). Thus, in a preferred embodiment of the present invention, the method for processing a fluid comprises the steps of:
(a) Providing a syringe body (1) filled with a fluid (11), wherein the plunger comprises a sealing plate (6) and a perforated sealing plate (7) at its lower end;
(b) Connecting a syringe closure (3) to the syringe body (1), in particular wherein the syringe closure (3) comprises at least one perforation cage (9);
(c) Separating the fluid into two or three phases by centrifugation or sedimentation, wherein the first phase reaches the syringe closure (3), the second phase remains in the syringe body (1) below the syringe plunger (2) and the optional third phase remains in the syringe body (1) between the perforated sealing plate (7) and the sealing plate (6);
(d) Removing the syringe closure (3) from the syringe body (1);
(i) Connecting the syringe body (1) to a container by a connector, in particular wherein the container is a second syringe comprising preferably a syringe plunger (2) according to the present invention and the first and second syringe are connected by a luer to luer connector;
(ii) Pushing or pulling the fluid of the syringe body (1) through the connector into the container;
(iii) Optionally, pushing or pulling the fluid back into the syringe body (1);
(iv) Removing the syringe body (1) from the container and the connector;
(b') Connecting a syringe closure (3) to the syringe body (1), in particular wherein the syringe closure (3) comprises at least one perforation cage (9); and
(c') Separating the fluid into two or three phases by centrifugation or sedimentation, wherein the first phase reaches the syringe closure (3), the second phase remains in the syringe body (1) below the syringe plunger (2) and the optional third phase remains in the syringe body (1) between the perforated sealing plate (7) and the sealing plate (6),
wherein preferably, steps ii) is repeated 1 to 10 times and step iii) is repeated 0 to 9 times.

The device, the syringe closure (3) and the method of processing a fluid according to the present invention are preferably for processing a fluid using centrifugal or gravitational force. The gravitational force e.g. for sedimentation is preferably of 0,9 to about 1,1 g, more preferably of about 1 g (normal acceleration of gravity). The centrifugal force for e.g. centrifugation is preferably of about 1 rcf to about 5000 rcf, of about 100 rcf to about 5000 rcf, or of about 400 to 3000 rcf for centrifugation. Thus, the device including the syringe body (1), the syringe plunger (2) the sealing plates (6 and 7), the syringe closure (3), and the perforation cages are preferably designed to withstand centrifugal or gravitational force as defined above, in particular if they are centrifuged in common centrifuges.

The device, the syringe closure (3) and the method of processing a fluid according to the present invention are preferably for the clean and lossless release (as further defined below) of individual phases of multiphase fluids after separation using centrifugal or gravitational forces.

The present invention also refers to a use of a device according to the present invention, a syringe plunger (2) according to the present invention, a syringe closure (3) according to the present invention or the method according to the present invention for processing a fluid into two, three or more phases. In particular, the present invention refers to a use of a device according to the present invention, a syringe plunger (2) according to the present invention, a syringe closure (3) according to the present invention or the method according to the present invention for the clean and lossless release of individual phases of multiphase fluids after separation using centrifugal or gravitational forces. If the device is used for separating a fluid into three phases, the present invention is in particular for the clean and lossless release of the second (middle) phase as defined herein. If the device is used for separating a fluid into two phases, the present invention is in particular for the clean and lossless release of the second phase (uppermost phase) as defined herein. A clean release means preferably that the individual phases can be separated from each other without any contamination of any phase with another phase. Thus, each individual phase (and in particular the second phase) can preferably be obtained with a purity of about 80 % to about 99%, of about 90% to about 99% or of about 95% to about 99%. It preferably also means that the individual phases can be obtained without any environmental contamination such as air, further fluids, viruses, fungi or bacteria. A lossless release means preferably that all separated phases can be released completely without remaining remains (apart from any adhesions in the device or in parts of the device). Thus, about 90 % to about 99%, more preferably 95% to about 99% of the volume of each individual phase can preferably be obtained by using the device according to the present invention.

In a preferred embodiment, the use is for processing (or separating) lipoaspirate and/or adipose tissue into a tumescent phase (first phase), a fat graft phase (second phase), and an oily phase (third phase). The use for processing lipoaspirate and/or adipose tissue is preferably for obtaining or providing a fat graft phase (second phase), which is preferably highly concentrated and ready for further use such as plastic surgery, lipofilling, transplantation or tissue regeneration.

The use may also comprise a praeprocessing of the fluid in the device according to the present invention e.g. by the addition of active components such as enzymes, e.g. for collagenase digestion, in particular of lipoaspirate.

In a further preferred embodiment of the present invention, the use is for processing blood, in particular whole blood, more particularly anticoagulated whole blood, into an erythrocytes phase (first phase) and a second phase comprising the buffy coat and the plasma (high volume PRP) or into an erythrocytes phase (first phase), highly concentrated PRP (second phase), and plasma (third phase).

In a further preferred embodiment of the present invention, the use is for enriching cells, in particular progenitor cells and/or stem cells, in a fat graft or bone marrow, for homogenizing a fat graft and/or for processing whole blood, preferably to produce PRP and/or PPP.

The use for processing blood is preferably for obtaining or providing highly concentrated PRP or high volume PRP from blood, in particular whole blood. The obtained or provided highly concentrated PRP or high volume PRP is preferably ready for further use such as transplantation or in combination with a fat graft phase in plastic surgery, lipofilling, transplantation or tissue regeneration.

In the following the invention is illustrated by the subsequent examples. These examples are to be considered as specific embodiments of the invention and shall not be considered to be limiting.

### Example 1 - Syringe system for processing lipoaspirate

One distinct preferred embodiment of a closed sterile device for processing a liquid or suspension is shown in Figures 1 to 5. The device comprises a syringe body (1), a specific syringe plunger (2) and a specific syringe closure (3). It is dimensioned, to fit in the most common centrifuge cups. The system is made from plastic. Like common syringe systems, the device is filled with lipoaspirate (11) by moving the syringe plunger (2, 15), which is shown in Figure 2B and Figure 5B. After filling the syringe system, the syringe closure (3) is connected (Figure 2C and Figure 5C). Subsequently, the constituents are separated by sedimentation or centrifugation. Centrifugation can for example be performed for 2 min at 1600 rcf.

To store the oily phase (14) after sedimentation, the syringe plunger has two different plates. The plate at the end of the plunger (7) shows 8 circular arranged and specific sized openings 0.3 mm diameter (10). Therefore, pressure on the plunger (16) leads to a passing of the sedimented oily phase (14) through the end plate (Figure 2D and Figure 5D). Due to the tissue-structure, the fat phase is not able to pass the openings and remains inside the syringe body but outside the plunger reservoir. Furthermore, the collagen-fibres of the fat tissue can seal the openings after pressure, to prevent the oily phase (14) to flow back into the syringe body (1). The plate (6) is a sealing plate and therefore does not have any openings. Hence, the stored oily phase (14) remains in this reservoir. As the perforated sealing plate (7) is movable within the syringe body (independently from any movement of the syringe plunger), up to *8 ml* can be stored in this reservoir. Above the sealing plate the end of the plunger is removeable, so that the system can fit in most centrifuges. To achieve this, a bayonet-catch is used.

The syringe closure (3) is connected to the syringe body at the most distal end over a male luer-lock connection (5) of the syringe body (1). The bottom of the closure is conical (Figure 1-2 and Figure 4-5). After the female luer (8), the syringe closure provides two different perforation cages (Figure 3; 9.1 and 9.2). There, the lipoaspirate (11) is filtered. The heavier tumescent (12) phase (fluid) can pass through the cage openings (19.1), which is shown in Figure 2E and Figure 5E. Furthermore, vertical cage openings (19.2) are added to the perforation cage. As shown in Figure 3 two perforation cages are used with oval openings (19.1, 19.2). The cage openings of both cages are oval (19.1, 19.2). Their size decreases from the inside cage 0.6 mm diameter (9.1) to the outside cage 0.2 mm diameter (9.2). In a further example, their size decreases from the inside cage 0.4 mm diameter (9.1) to the outside cage 0.1 mm diameter (9.2). However, the tissue-structure prevents the fat-graft from passing the perforation cages. The collagen fibres can seal these openings. Therefore, nearly no volume loss of the fat-phase is observed. Subsequently, the tumescent phase in the syringe closure can be rejected and the sterile processed fat can be transplanted by pressing (17) the syringe plunger as usual (Figure 2F and Figure 5F). The male luer-lock connection is configurated to receive a lipofilling cannula, a syringe needle, a connector, or a three-way stop cock. To protect the luer-lock connection during centrifugation a ring contacts both, the closure and the syringe body (18), absorbing the applied centrifugal force.

### Example 2 - Cell Enriched Lipo-Transfer (CELT)

The fat phase of lipoaspirate obtained as disclosed in Example 1 is homogenized. Therefore, the syringe closure is removed and a 180 degrees luer-lock to luer-lock connector with a variable inner diameter of 1.2mm is connected. On the other side of this connector an empty syringe body (1) with a syringe plunger (2) is connected. The fat is pushed from syringe to syringe eleven times. After the homogenization an empty syringe closure (3) is added and fat is centrifuged again at 1600 rcf for 2 min. As described above, the movable perforated sealing plate (7) is pushed due to centrifugal force through the oily phase, so the oily phase is located between the sealing plate (6) and the movable perforated sealing plate (7). The cytosol/remaining tumescent phase is pushed through the perforation cages (9) to the syringe closure (3). The remaining fat-phase with concentrated stem cells if directly ready for further use as e.g. injection into the patient from which the fluid/sample was obtained.

### Example 3 - Processing of anticoagulated blood whole blood

To produce high volume PRP, a mixture of 1.4 ml anticoagulance citrate buffer and 8.6 mL whole blood is provided in a syringe system (as shown in Fig. 1) comprising a syringe plunger (2) having a sealing plate (6) and a movable perforated sealing plate (7). The perforated plate is movable along the plunger's axis. The movable perforated plate is manufactured that its density ranges from 1.05 to 1.09 g/ml and that its openings allow the passage of red blood cells but not platelets. The syringe closure is each attached and the anticoagulated blood in the syringe is centrifuged at 900 rcf for 5 minutes. In the centrifuge the syringe is placed with the finger flange (4) at the bottom of the centrifuge container. After the centrifugation the reservoir between the movable perforated sealing plate (7) and the sealing plate (6) contains the red blood cell phase. The PRP phase remains between the movable perforated sealing plate (7) and syringe opening (22). Performing pressure to the plunger, the concentrated platelets are ready for further use. For example, they can be released to either another syringe connected via a female-to-female Luer-connector or after attachment of a cannula to the site of tissue regeneration.

### Example 4 - Processing of anticoagulated blood whole blood

To produce high concentration PRP, a mixture of 1.4 ml anticoagulance citrate buffer and 8.6 mL whole blood is provided in a syringe system (as shown in Fig. 1) comprising a syringe plunger (2) having a sealing plate (6) and a movable perforated sealing plate (7) and a syringe closure (3), comprising at least one perforation cage (9). The perforation cages openings allow the passage of red blood cells but not platelets. The syringe is placed in a centrifuge with the syringe closure at the bottom of the centrifuge container. The anticoagulated blood in the syringe is centrifuged at 900 rcf for 5 minutes. The red blood cell phase is in the syringe closure and can be discarded. The platelets are close to the syringe opening (22) and are ready for further use. For example, they can be released to either another syringe connected via a female-to-female Luer-connector or after attachment of a cannula to the site of tissue regeneration. The platelets can be released with a variable plasma volume and therefore used in a desired concentration.

## Claims

1. A device for processing a fluid by centrifugation and/or sedimentation comprising:
(i) a syringe body (1),
(ii) a syringe plunger (2) comprising a sealing plate (6) and a perforated sealing plate (7) at the lower end of the plunger, wherein the perforated sealing plate (7) is movably attached to the syringe plunger (2) and the distance between the movable perforated sealing plate (7) and the sealing plate (6) is variable, and
(iii) a syringe closure (3), comprising at least one perforation cage (9), an opening (8) and a collection cup (21), wherein the perforation cage (9) is between the opening (8) and the collection cup (21) and wherein the perforation cage (9) is configured in that any liquid passing from the opening (8) to the collection cup (21) has to pass said at least one perforation cage (9).

2. The device according to claim 1, wherein the syringe plunger comprises a lower part comprising the sealing plate (6) and the perforated sealing plate (7) and an upper part and wherein the upper part of the syringe plunger is removable from the lower part of the syringe plunger.

3. The device according to any one of claims 1 or 2, wherein the perforated sealing plate (7) comprises at least 2 perforations or openings (10), more preferably about 2 to 16 perforations or openings, in particular wherein the openings (10) are circular and have a diameter of about 0.2 µm to about 0.8 mm or wherein the openings (10) are oval, triangular, rectangular, square or multi-sided and have a perimeter of about 0.6 µm to about 2.5 mm.

4. The device according any one of the preceding claims, wherein the perforation cage is formed by a net, a linear perforation plate, a membrane, or a cylindrical perforation cage.

5. The device according to any one of claim 1 to 3, wherein the syringe closure comprises 2, 3, or 4 perforation cages.

6. The device according to any one of the preceding claims, wherein each perforation cage has at least two vertical cage openings (19.2), more preferably 2 to 16 vertical cage openings, in particular wherein at least one perforation cage has at least one horizontal cage openings (19.1), more preferably about 1 to 12 horizontal cage openings (19.1).

7. The device according to claim 6, wherein the cage openings (19.1, 19.2, 20.1 and/or 20.2) are circular and have a diameter of about 0.2 µm to about 0.8 mm or wherein the cage openings are oval, triangular, rectangular, square or multi-sided and have a perimeter of about 0.6 µm to about 2.5 mm.

8. A method of processing a fluid, the method comprises the steps of:
(a) Providing a syringe body (1) filled with a fluid (11), wherein the plunger comprises a sealing plate (6) and a perforated sealing plate (7) at its lower end, wherein the perforated sealing plate (7) is movably attached to the syringe plunger (2) and the distance between the movable perforated sealing plate (7) and the sealing plate (6) is variable;
(b) Connecting a syringe closure (3) to the syringe body (1), wherein the syringe closure (3) comprises at least one perforation cage (9) an opening (8) and a collection cup (21), wherein the perforation cage (9) is between the opening (8) and the collection cup (21) and wherein the perforation cage (9) is configured in that any liquid passing from the opening (8) to the collection cup (21) has to pass said at least one perforation cage (9); and
(c) Separating the fluid into two, three or more phases by centrifugation or sedimentation.

9. The method according to claim 8, wherein the method comprises additionally the steps of:
(i) Connecting the syringe body (1) to a container by a connector, preferably by a luer to luer connector; and
(ii) Pushing or pulling the fluid of the syringe body (1) through the connector into the container; and optionally
(iii) Pushing or pulling the fluid back into the syringe body (1).

10. The method according to claim 9, wherein step (ii) is repeated 1 to 10 times and step (iii) is repeated 0 to 9 times.

11. The method according to any one of claims 9 or 10, wherein steps (i) to (iii) are performed subsequent to step (c) or wherein steps (i) to (iii) are performed subsequent to step (a) and prior to step (b).

12. Use of a device according to any one of claims 1 to 7 or the method according to any one of claims 8 to 11 for the clean and lossless release of individual phases of multiphase fluids after separation using centrifugal or gravitational forces, in particular for separating a fluid such as lipoaspirate, blood, a fat graft or bone marrow into two, three or more phases, and/or for enriching cells, in particular progenitor cells and/or stem cells, in a fat graft or bone marrow, and/or for homogenizing a fat graft and/or for processing whole blood, preferably to produce PRP.

## Patentansprüche

1. Eine Vorrichtung zur Verarbeitung eines Fluids durch Zentrifugation und/oder Sedimentation, umfassend:
(i) einen Spritzenkörper (1),
(ii) einen Spritzenkolben (2), der eine Dichtungsplatte (6) und einer perforierten Dichtungsplatte (7) am unteren Ende des Kolbens umfasst, wobei die perforierte Dichtungsplatte (7) beweglich am Spritzenkolben (2) befestigt ist und der Abstand zwischen der beweglichen perforierten Dichtungsplatte (7) und der Dichtungsplatte (6) variabel ist, und
(iii) einen Spritzenverschluss (3), der mindestens einen Perforationskäfig (9), eine Öffnung (8) und einen Auffangbehälter (21) umfasst, wobei sich der Perforationskäfig (9) zwischen der Öffnung (8) und dem Auffangbehälter (21) befindet und wobei der Perforationskäfig (9) so konfiguriert ist, dass jede Flüssigkeit, die von der Öffnung (8) zum Auffangbehälter (21) gelangt, den mindestens einen Perforationskäfig (9) passieren muss.

2. Die Vorrichtung nach Anspruch 1, wobei der Spritzenkolben einen unteren Teil, der die Dichtungsplatte (6) und die perforierte Dichtungsplatte (7) umfasst, und einen oberen Teil umfasst, und wobei der obere Teil des Spritzenkolbens vom unteren Teil des Spritzenkolbens entfernbar ist.

3. Die Vorrichtung nach einem der Ansprüche 1 oder 2, wobei die perforierte Dichtungsplatte (7) mindestens 2 Perforationen oder Öffnungen (10), vorzugsweise etwa 2 bis 16 Perforationen oder Öffnungen, umfasst, insbesondere wobei die Öffnungen (10) kreisförmig sind und einen Durchmesser von etwa 0,2 µm bis etwa 0,8 mm aufweisen oder wobei die Öffnungen (10) oval, dreieckig, rechteckig, quadratisch oder vielseitig sind und einen Umfang von etwa 0,6 µm bis etwa 2,5 mm aufweisen.

4. Die Vorrichtung nach einem der vorhergehenden Ansprüche, wobei der Perforationskäfig durch ein Netz, eine lineare Perforationsplatte, eine Membran oder einen zylindrischen Perforationskäfig gebildet wird.

5. Die Vorrichtung nach einem der Ansprüche 1 bis 3, wobei der Spritzenverschluss 2, 3 oder 4 Perforationskäfige umfasst.

6. Die Vorrichtung nach einem der vorhergehenden Ansprüche, wobei jeder Perforationskäfig mindestens zwei vertikale Käfigöffnungen (19.2), vorzugsweise 2 bis 16 vertikale Käfigöffnungen, aufweist, insbesondere wobei mindestens ein Perforationskäfig mindestens eine horizontale Käfigöffnung (19.1), vorzugsweise etwa 1 bis 12 horizontale Käfigöffnungen (19.1), aufweist.

7. Die Vorrichtung nach Anspruch 6, wobei die Käfigöffnungen (19.1, 19.2, 20.1 und/oder 20.2) kreisförmig sind und einen Durchmesser von etwa 0,2 µm bis etwa 0,8 mm haben oder wobei die Käfigöffnungen oval, dreieckig, rechteckig, quadratisch oder vielseitig sind und einen Umfang von etwa 0,6 µm bis etwa 2,5 mm haben.

8. Ein Verfahren zur Verarbeitung eines Fluids, wobei das Verfahren die folgenden Schritte umfasst:
(a) Bereitstellen eines mit einem Fluid (11) gefüllten Spritzenkörpers (1), wobei der Kolben an seinem unteren Ende eine Dichtungsplatte (6) und eine perforierte Dichtungsplatte (7) umfasst, wobei die perforierte Dichtungsplatte (7) beweglich am Spritzenkolben (2) befestigt ist und der Abstand zwischen der beweglichen perforierten Dichtungsplatte (7) und der Dichtungsplatte (6) variabel ist;
(b) Verbinden eines Spritzenverschlusses (3) mit dem Spritzenkörper (1), wobei der Spritzenverschluss (3) mindestens einen Perforationskäfig (9), eine Öffnung (8) und einen Auffangbehälter (21) umfasst, wobei sich der Perforationskäfig (9) zwischen der Öffnung (8) und dem Auffangbehälter (21) befindet und wobei der Perforationskäfig (9) so konfiguriert ist, dass jede Flüssigkeit, die von der Öffnung (8) zum Auffangbehälter (21) gelangt, den mindestens einen Perforationskäfig (9) passieren muss; und
(c) Trennen des Fluids in zwei, drei oder mehr Phasen durch Zentrifugieren oder Sedimentieren.

9. Das Verfahren nach Anspruch 8, wobei das Verfahren zusätzlich die folgenden Schritte umfasst:
(i) Verbinden des Spritzenkörpers (1) mit einem Behälter mittels eines Verbindungsstücks, vorzugsweise mittels eines Luer-Luer-Verbindungsstücks; und
(ii) Drücken oder Ziehen des Fluids aus dem Spritzenkörper (1) durch das Verbindungsstück in den Behälter; und optional
(iii) Drücken oder Ziehen der Fluids zurück in den Spritzenkörper (1).

10. Das Verfahren nach Anspruch 9, wobei Schritt (ii) 1 bis 10 Mal wiederholt wird und Schritt (iii) 0 bis 9 Mal wiederholt wird.

11. Das Verfahren nach einem der Ansprüche 9 oder 10, wobei die Schritte (i) bis (iii) nach Schritt (c) durchgeführt werden oder wobei die Schritte (i) bis (iii) nach Schritt (a) und vor Schritt (b) durchgeführt werden.

12. Verwendung einer Vorrichtung gemäß einem der Ansprüche 1 bis 7 oder des Verfahrens gemäß einem der Ansprüche 8 bis 11 zur sauberen und verlustfreien Freisetzung einzelner Phasen von mehrphasigen Fluiden nach der Trennung unter Verwendung von Zentrifugal- oder Gravitationskräften, insbesondere zum Trennen eines Fluids wie Lipoaspiration, Blut, eines Fetttransplantats oder Knochenmarks in zwei, drei oder mehr Phasen und/oder zum Anreichern von Zellen, insbesondere Vorläuferzellen und/oder Stammzellen, in einem Fetttransplantat oder Knochenmark und/oder zum Homogenisieren eines Fetttransplantats und/oder zur Verarbeitung von Vollblut, vorzugsweise zur Herstellung von PRP.

## Revendications

1. Dispositif pour traiter un fluide par centrifugation et/ou sédimentation comprenant :
(i) un corps de seringue (1),
(ii) un piston de seringue (2) comprenant une plaque d'étanchéité (6) et une plaque d'étanchéité perforée (7) au niveau de l'extrémité inférieure du piston, dans lequel la plaque d'étanchéité perforée (7) est fixée, de manière mobile, au piston de seringue (2) et la distance entre la plaque d'étanchéité perforée (7) mobile et la plaque d'étanchéité (6) est variable, et
(iii) une fermeture de seringue (3) comprenant au moins une cage de perforation (9), une ouverture (8) et une coupelle de collecte (21), dans lequel la cage de perforation (9) est entre l'ouverture (8) et la coupelle de collecte (21) et dans lequel la cage de perforation (9) est configurée de sorte que tout liquide passant de l'ouverture (8) à la coupelle de collecte (21) doit passer par ladite au moins une cage de perforation (9).

2. Dispositif selon la revendication 1, dans lequel le piston de seringue comprend une partie inférieure comprenant la plaque d'étanchéité (6) et la plaque d'étanchéité perforée (7) et une partie supérieure et dans lequel la partie supérieure du piston de seringue peut être retirée de la partie inférieure du piston de seringue.

3. Dispositif selon l'une quelconque des revendications 1 ou 2, dans lequel la plaque d'étanchéité perforée (7) comprend au moins 2 perforations ou ouvertures (10), encore de préférence environ de 2 à 16 perforations ou ouvertures, en particulier dans lequel les ouvertures (10) sont circulaires et ont un diamètre d'environ 0,2 µm à environ 0,8 mm ou bien dans lequel les ouvertures (10) sont ovales, triangulaires, rectangulaires, carrées ou à plusieurs côtés et ont un périmètre d'environ 0,6 µm à environ 2,5 mm.

4. Dispositif selon l'une quelconque des revendications précédentes, dans lequel la cage de perforation est formée par un filet, une plaque de perforation linéaire, une membrane ou une cage de perforation cylindrique.

5. Dispositif selon l'une quelconque des revendications 1 à 3, dans lequel la fermeture de seringue comprend 2, 3 ou 4 cages de perforation.

6. Dispositif selon l'une quelconque des revendications précédentes, dans lequel chaque cage de perforation a au moins deux ouvertures de cage verticales (19.2), encore de préférence de 2 à 16 ouvertures de cage verticales, en particulier dans lequel au moins une cage de perforation a au moins une ouverture de cage horizontale (19.1), encore de préférence environ de 1 à 12 ouvertures de cage horizontales (19.1).

7. Dispositif selon la revendication 6, dans lequel les ouvertures de cage (19.1, 19.2, 20.1 et/ou 20.2) sont circulaires et ont un diamètre d'environ 0,2 µm à environ 0,8 mm ou bien dans lequel les ouvertures de cage sont ovales, triangulaires, rectangulaires, carrées ou à plusieurs côtés et ont un périmètre d'environ 0,6 µm à environ 2,5 mm.

8. Méthode pour traiter un fluide, la méthode comprenant les étapes consistant à :
(a) prévoir un corps de seringue (1) rempli avec un fluide (11), dans laquelle le piston comprend une plaque d'étanchéité (6) et une plaque d'étanchéité perforée (7) au niveau de son extrémité inférieure, dans laquelle la plaque d'étanchéité perforée (7) est fixée, de manière mobile, au piston de seringue (2) et la distance entre la plaque d'étanchéité perforée (7) mobile et la plaque d'étanchéité (6) est variable ;
(b) raccorder une fermeture de seringue (3) au corps de seringue (1), dans laquelle la fermeture de seringue (3) comprend au moins une cage de perforation (9), une ouverture (8) et une coupelle de collecte (21), dans laquelle la cage de perforation (9) est entre l'ouverture (8) et la coupelle de collecte (21) et dans laquelle la cage de perforation (9) est configurée de sorte que tout liquide passant de l'ouverture (8) à la coupelle de collecte (21) doit passer par ladite au moins une cage de perforation (9) ; et
(c) séparer le fluide en deux, trois phases ou plus par centrifugation ou sédimentation.

9. Méthode selon la revendication 8, dans laquelle la méthode comprend de plus les étapes consistant à :
(i) raccorder le corps de seringue (1) à un récipient par un connecteur, de préférence par un connecteur Luer - Luer ; et
(ii) pousser ou extraire le fluide du corps de seringue (1) par le biais du connecteur dans le récipient ; et facultativement
(iii) pousser ou extraire le fluide à nouveau dans le corps de seringue (1).

10. Méthode selon la revendication 9, dans laquelle l'étape (ii) est répétée de 1 à 10 fois et l'étape (iii) est répétée de 0 à 9 fois.

11. Méthode selon l'une quelconque des revendications 9 ou 10, dans laquelle les étapes (i) à (iii) sont réalisées suite à l'étape (c) ou bien dans laquelle les étapes (i) à (iii) sont réalisées suite à l'étape (a) et avant l'étape (b).

12. Utilisation d'un dispositif selon l'une quelconque des revendications 1 à 7 ou bien méthode selon l'une quelconque des revendications 8 à 11 pour la libération propre et sans perte de phases individuelles de fluides à plusieurs phases après la séparation en utilisant les forces centrifuges ou gravitationnelles, en particulier pour séparer un fluide tel qu'un tissu aspiré par liposuccion, du sang, un greffon de tissu adipeux ou de la moelle osseuse en deux, trois phases ou plus, et/ou pour enrichir des cellules, en particulier des cellules progénitrices et/ou des cellules souches, dans un greffon de tissu adipeux ou de la moelle osseuse, et/ou pour homogénéiser un greffon de tissu adipeux et/ou pour traiter du sang total, de préférence pour produire du PRP.
